# EUROPEAN PATENT APPLICATION

(11) **EP 4 257 082 A1**
(43) Date of publication of application: **11.10.2023**
(21) Application number: 21900669.9
(22) Date of filing: 02.12.2021
(51) Int. Cl.: A61F 2/24

(54) **ANNULOPLASTY RING AND METHOD FOR PRODUCING SAME**

(30) Priority: 02.12.2020 JP 2020200594
(71) Applicant: S.H.I Bio Co., Ltd., Kyoto, 619-0237 (JP)
(72) Inventor: ITO, Toshiaki, Soraku-gun, Kyoto 619-0237 (JP)
(74) Representative: Weickmann & Weickmann PartmbB
(86) International application number: PCT/JP2021/044299
(87) International publication number: WO 2022/118919

(57) **Abstract**

The present invention relates to an annuloplasty ring having a core part 10 and an outer layer 20. The core part 10 is curved such that a band-shaped plate member made of a spring material defines an opening part in a shape along a mitral valve annulus and presents a C-shape. The outer layer 20 is a layer made of a polymer material and covering a surface of the aforementioned core part 10. A longitudinal axis 10a of the cross section of the aforementioned band-shaped plate member when the core part is cut along a flat plane containing the central axis Z passing through the opening part in the blood flow direction F is inclined with respect to the central axis Z, and the inclination is such that the diameter of the opening part decreases as the central axis Z moves in the blood flow direction F.

## Description

### [Technical Field]

The present invention relates to annuloplasty rings used in annuloplasty for the tricuspid valve and mitral valve of the heart, and production methods thereof.

### [Background Art]

Conventionally, in valvuloplasty for treating various valve diseases in the heart (especially diseases related to the valve annulus) such as mitral regurgitation, various annuloplasty rings (also called artificial valve annulus, valvuloplasty band, etc.) have been used to correct and reinforce the valve annulus (e.g., Patent Literature 1, Patent Literature 2). Hereinafter, the annuloplasty ring is also to be simply referred to as "a ring".

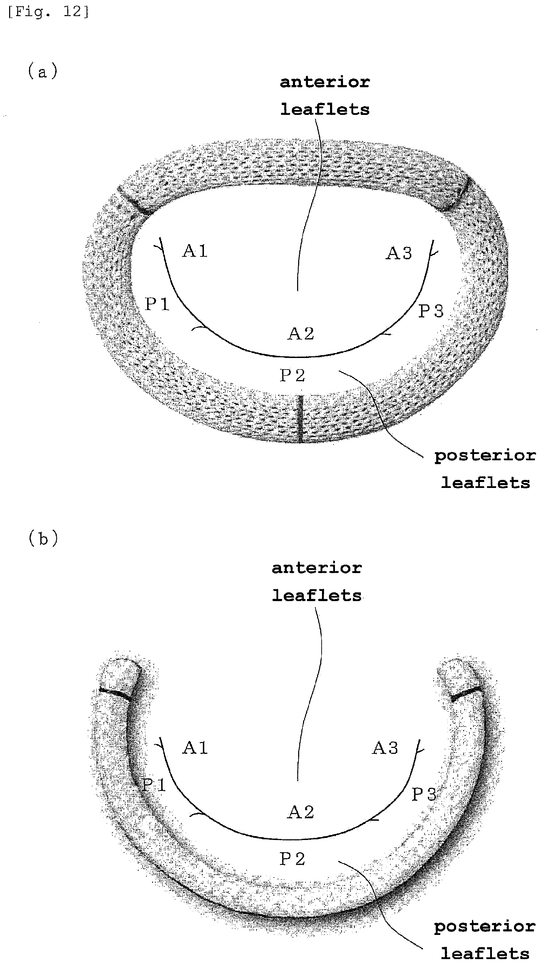

The shape of the ring described in Patent Literature 1 is, as shown in an example of a commercially available product in Fig. 12(a), a closed loop shape that completely encircles the orifice of the mitral valve, and has a three-dimensional shape that partially undulates in the blood flow direction (a shape called a saddle shape, etc.). This ring has a core part with a closed loop shape and a polyester cloth covering the outer surface of the core. In the example of Fig. 12(a), the core has a composite structure in which metal and plastic are laminated. The core part imparts moderate rigidity to maintain the shape of the entire ring, and partially imparts flexibility according to the biological movement of the mitral valve.

On the other hand, the annuloplasty ring described in Patent Literature 2 belongs to the concept of a ring, but is a C-shape (partial ring shape) with a part of the closed loop shape notched as in the example of a commercially available mitral annuloplasty ring shown in Fig. 12(b), and is not a three-dimensional shape like a saddle shape but a shape that forms a C shape in a flat plane. This ring also has a core and a polyester cloth covering the outer surface of the core, but the core is made of flexible silicone rubber, and due to the flexibility thereof, it is possible to follow the three-dimensional movement of the mitral valve annulus without deforming the original saddle shape of the mitral valve annulus.

### [Citation List]

### [Patent Literature]

[PTL 1]
   US-2009-0177276A1
[PTL 2]
   US Patent No. 4055861

### [Summary of Invention]

### [Technical Problem]

However, the present inventors examined in detail the above-mentioned conventional annuloplasty rings and found the following problems.

First, problems common to both the closed loop-shaped ring shown in Fig. 12(a) and the C-shaped ring shown in Fig. 12(b) are as follows.

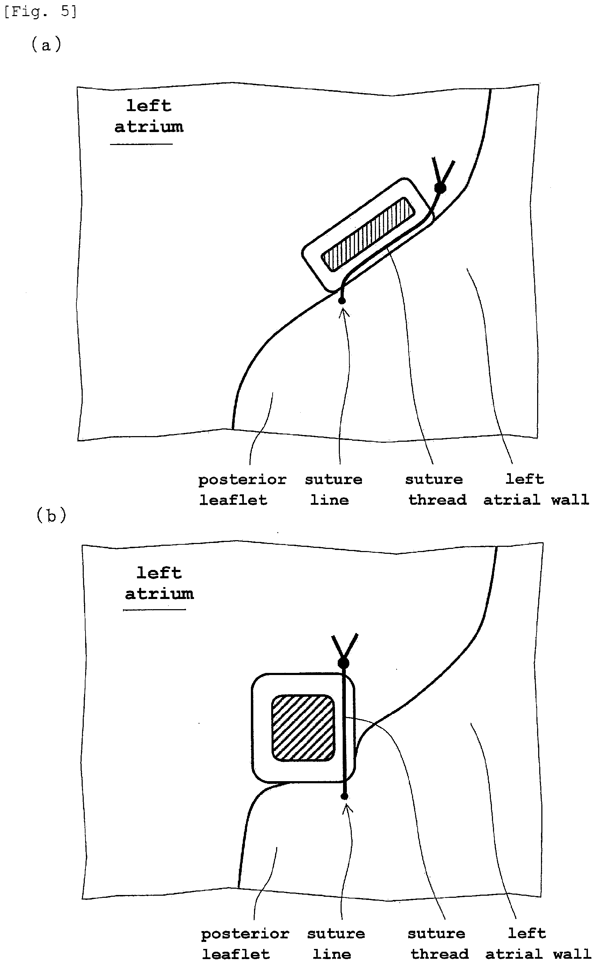

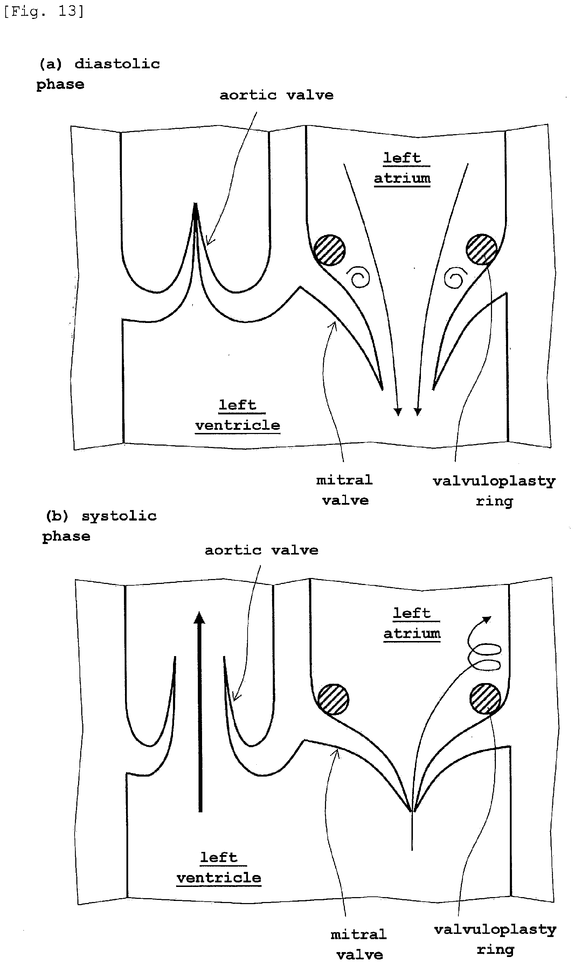

For example, in the ring used for mitral annuloplasty, the cross-sectional shape of the ring (the cross-sectional shape when the ring is cut along a flat plane containing the central axis of the ring) is, as shown in Figs. 13(a) and 13(b), circular or nearly square (a square with rounded corners), which makes it difficult to achieve close contact with the surface of the mitral valve with a sufficiently large contact area. These rings, as also shown in Fig. 5(b), protrude relatively large from the surface of the mitral valve annulus into the flow path on the left atrium side. Therefore, as shown in Fig. 13(a), the resistance to blood flow is large and turbulent flow is unpreferably caused. In addition, as shown in Fig. 13(b), even with mild blood regurgitation that is free of hemodynamical problems, a turbulent flow occurs when the regurgitant jet hits the ring, possibly resulting in severe hemolytic anemia.

Problems inherent to the closed loop shaped ring shown in Fig. 12(a) have been found to include the following (the problem is described taking mitral annuloplasty as an example, but the same is true in the case of tricuspid annuloplasty).
(a) The base of the anterior leaflet of the mitral valve is fixed to a ring with relatively high rigidity, which inhibits leaflet motion.
(b) Since the orifice area of the mitral valve is limited to the opening area (immovable) of the ring, for example, the valve orifice area becomes insufficient when motion is applied, problematically resulting in a stenotic state.
(c) It is necessary to hang the fixing thread on the outer layer while avoiding the metal core in the ring. As also shown in Fig. 5(b), the ring is located on the downstream side of the suture line, making the ring protrude into the mitral valve orifice, and the knot of the fixing thread protrudes into the flow path.

Also, problems inherent to the C-shaped flexible annuloplasty ring shown in Fig. 12(b) have been found to include the following.
(d) Having a flexible cord state, a force from the valve annulus repeatedly acts particularly on the end part (two ends facing each other across the C-shaped notch part), a large tension repeatedly acts on the fixing thread at the end part, damaging the fixing thread and valve annulus tissue.
(e) Due to the repeated action of tension on the fixing thread at the end, the process of microcleft formation and repair of the valve annulus tissue is repeated, and the ring may be displaced from the initial sewing position.

The present invention aims to provide an annuloplasty ring capable of reducing the above-mentioned problems, and to provide production methods of the annuloplasty ring.

### [Solution to Problem]

The main constitutions of the present invention are as follows.
[1] An annuloplasty ring comprising a core part and an outer layer made of a polymer material, wherein
   the core part has a curved structure in which a band-shaped plate member made of a spring material defines an opening part in a shape along a mitral valve annulus and presents a C-shape,
   the outer layer covers a surface of the aforementioned core part,
   the longitudinal axis of the cross section of the aforementioned band-shaped plate member when the core part is cut along a flat plane containing the central axis passing through the aforementioned opening part in the blood flow direction is inclined with respect to the aforementioned central axis, and
   the inclination is such that the diameter of the aforementioned opening part decreases as the aforementioned central axis moves in the blood flow direction.
[2] The annuloplasty ring of the aforementioned [1], wherein the annuloplasty ring is a mitral annuloplasty ring for application to a mitral valve annulus.
[3] The annuloplasty ring of the aforementioned [2], wherein the band-shaped plate member is curved so as to present a C-shape while forming a saddle shape, a shape along the mitral valve annulus.
[4] The annuloplasty ring of any of the aforementioned [1] to [3], wherein the material of the band-shaped plate member is an Ni-Ti alloy which is a shape memory alloy.
[5] The annuloplasty ring of the aforementioned [4], wherein the above-mentioned Ni-Ti alloy has an Af point of not more than 15°C.
[6] The annuloplasty ring of any of the aforementioned [1] to [5], wherein the longitudinal axis of the above-mentioned cross section of the band-shaped plate member is inclined at an elevation angle of 45 to 60 degrees with respect to the above-mentioned flat plane perpendicular to the central axis.
[7] The annuloplasty ring of any of the aforementioned [1] to [6], wherein the outer layer is a layer made of cloth, the cloth layer is a braid tube obtained by weaving threads around a core material having the same cross-sectional shape as the above-mentioned cross-sectional shape of the above-mentioned band-shaped plate member.
[8] The annuloplasty ring of any of the aforementioned [1] to [7], which is used for treating a heart valve disease.
[9] A method for producing the annuloplasty ring of any of the aforementioned [1] to [8], the method comprising,
   (i) a core part formation step that includes preparing an arc-shaped member for forming the core part of the aforementioned annuloplasty ring, and applying an external force to the arc-shaped member to cause deformation of the core part into a shape of the band-shaped plate member, and heat treating the aforementioned deformed shape of the band-shaped plate member to form a core part having the shape of the band-shaped plate member as an original shape, wherein
      the aforementioned arc-shaped member is a band-shaped plate having the same material, the same thickness, and the same width as the band-shaped plate member of the core part of the aforementioned annuloplasty ring, and is curved to form an arc, the plate surface of the band-shaped plate is flat, and the band-width direction of the band-shaped plate coincides with the radial direction of the aforementioned arc, and
   (ii) an outer layer formation step which is a step of covering a surface of the aforementioned core part with an outer layer made of a polymer material.
[10] The production method of the aforementioned [9], wherein the aforementioned arc-shaped member is a member curved to form an arc with a central angle of 135 to 225 degrees.
[11] The production method of the aforementioned [9] or [10], wherein the material of the above-mentioned band-shaped plate member is an Ni-Ti alloy which is a shape memory alloy, and
   the above-mentioned heat treatment is rapid cooling from the shape memory heat treatment temperature of the Ni-Ti alloy to a temperature lower than the Af point.
[12] The production method of the aforementioned [11], wherein the above-mentioned annuloplasty ring to be produced by the production method is a mitral annuloplasty ring for application to a mitral valve annulus,
   a core part of the mitral annuloplasty ring to be produced is curved such that the band-shaped plate member presents a C-shape while forming a saddle shape, a shape along the mitral valve annulus,
   each of two end parts of the above-mentioned arc-shaped member is provided with a through-hole for fixing the arc-shaped member in a state of being deformed into the aforementioned C-shape,
   in the core part formation step of the above-mentioned (i) in the production method, a jig is used to hold the aforementioned arc-shaped member deformed into the aforementioned C-shape and to perform a heat treatment in that state,
   the jig has a base part with a first flat plane, and a ring-fixing member is provided at a predetermined position on the first flat plane,
   the ring-fixing member has two inclined planes for fixing the aforementioned two end parts of the arc-shaped member that is deformed into a C-shape while forming the aforementioned saddle shape,
   the aforementioned two inclined planes are located at two positions on the first flat plane such that the two end parts of the aforementioned arc-shaped member are along the saddle shape,
   the aforementioned two inclined planes have the aforementioned inclination of the core part,
   the aforementioned two inclined planes have a fixing part for fixing the aforementioned two end parts at a height position corresponding to the aforementioned saddle shape,
   at predetermined positions on the first flat plane, three or more guide members protrude along the C-shape so as to maintain the state of the aforementioned arc-shaped member deformed into the C-shape forming the aforementioned saddle shape,
   two out of the aforementioned three or more guide members are each located inside the two opposing curved parts that curve at the greatest curvature in the aforementioned C-shape, and the two guide members each have a part inclined to the outside of the aforementioned curved part so as to incline the aforementioned arc-shaped member toward the inclined plane of the aforementioned core part, and
   the remaining guide members among the aforementioned three or more guide members are members that are positioned outside a central curved part connecting the aforementioned two opposing curved parts to prevent the aforementioned arc-shaped member from expanding outward, and restrict the shape of the arc-shaped member to the C-shape of the core part of the mitral annuloplasty ring to be produced.
[13] The production method of any of the aforementioned [9] to [12], wherein the outer layer is a layer made of cloth, the cloth layer is a braid tube obtained by weaving threads around a core material having the same cross-sectional shape as the above-mentioned cross-sectional shape of the above-mentioned band-shaped plate member.

### [Advantageous Effects of Invention]

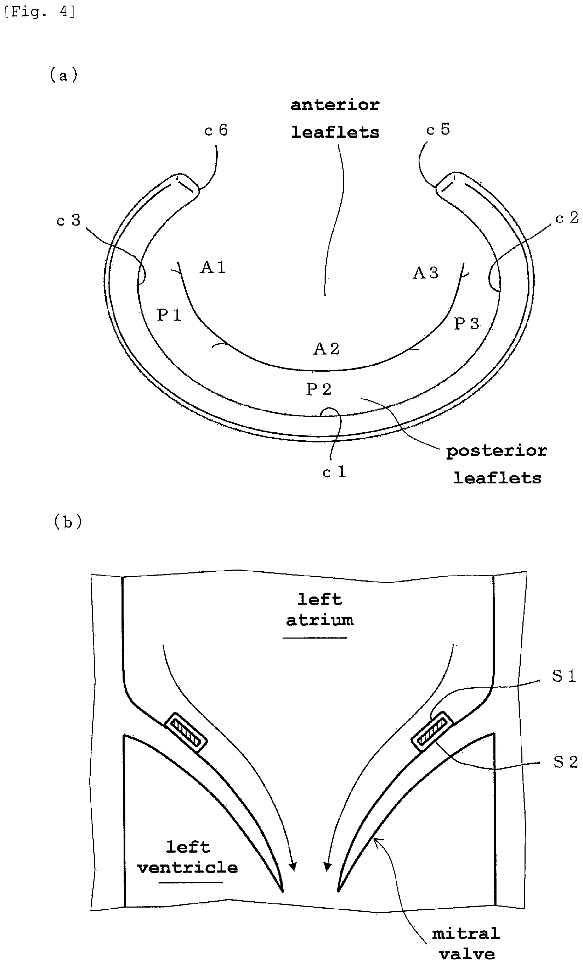

The annuloplasty ring according to the present invention (hereinafter also to be referred to as said ring) can be sewn snugly and more stably to the surface of the valve annulus (e.g., mitral valve annulus) of a patient or target animal with a larger contact area than that of conventional products. In addition, due to its own flat cross-sectional shape, the height of protrusion of said ring from the surface of the patient's valve annulus into the flow path is lower than in conventional products, as shown in Fig. 4(b), Fig. 5(a), thereby causing less turbulence in the blood flow.

As shown in Fig. 5(a), moreover, the bottom of the outer layer of said ring's own wide contact surface can be used as a passage pathway for a fixing thread, and the knot of the fixing thread can be made outside the outer circumference of said ring. In this sewing embodiment, as shown in Fig. 5(a), the fixing thread enters the outer layer on the opening side of said ring from the position of the usual suture line, passes through the outer layer on the cardiac wall side (contact surface side), and exits from the outer circumference side of the outer layer to make a knot. Therefore, the present invention suppresses the minute turbulence that conventionally occurs in blood flow due to the knots of fixing threads.

With the above-mentioned effects, the problem of conventional products that turbulence in the blood flow is generated near the entrance of the mitral valve, and the like can be suppressed, and the possibility of blood coagulation and thrombus formation can be reduced.

### [Brief Description of Drawings]

[Fig. 1]

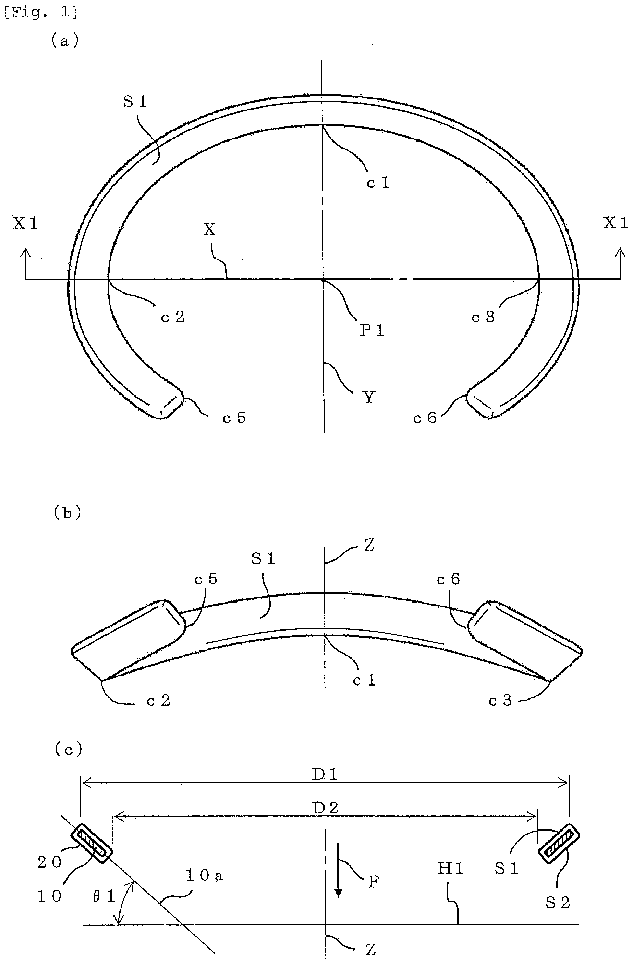

   Fig. 1 shows one example of the constitution of the annuloplasty ring according to the present invention. In Fig. 1(a), said ring is viewed along the central axis. The central axis is an axis that passes through the point P1 perpendicularly to the plane of paper in Fig. 1(a). In Fig. 1(b), said ring shown in Fig. 1(a) is viewed from the downward of Fig. 1(a). Fig. 1(c) is an arrow view along X1-X1 cut end surface in Fig. 1(a), and schematically shows the section of the core part and the outer layer. The cut end surface of the core part is hatched in order to distinguish regions, and hatching of the cut end surface of the outer layer is omitted.
[Fig. 2]

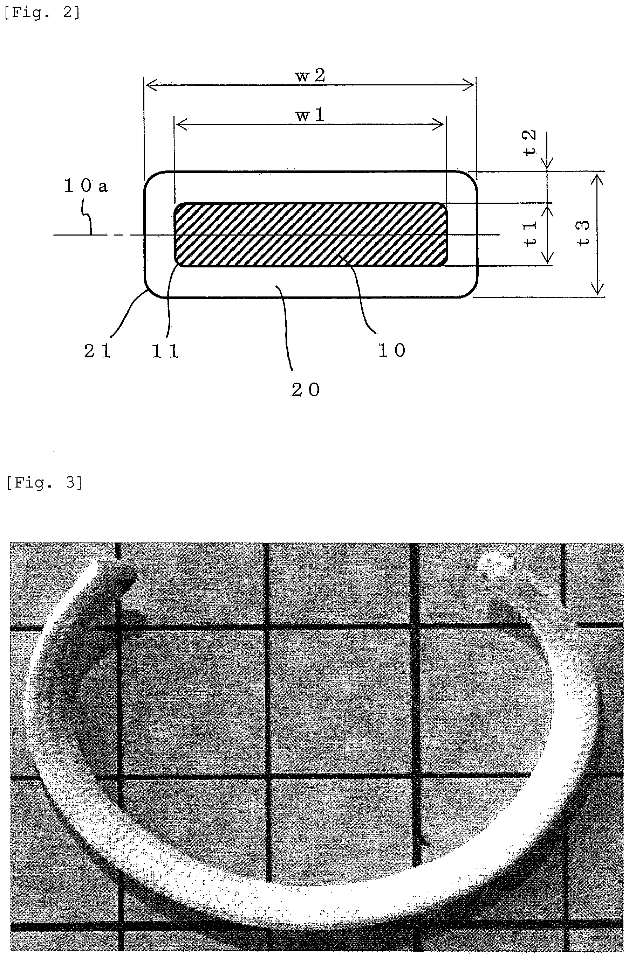

   Fig. 2 schematically shows section of the annuloplasty ring according to the present invention.
[Fig. 3]
   Fig. 3 is a photographic view showing one example of the external appearance of the example product of the annuloplasty ring according to the present invention, in which said ring is viewed along the central axis Z shown in Fig. 1(b). In the figure, said ring is placed on a surface with a grid of 10 mm on each side so that the size of the ring can be easily known.
[Fig. 4]
   Fig. 4 is a schematic diagram showing the state in which the annuloplasty ring according to the present invention is sewn on the mitral valve annulus. In Fig. 4(a), said ring is viewed along the central axis Z shown in Fig. 1(b), and anterior leaflets (A1, A2, A3) and posterior leaflets (P1, P2, P3) have been added to show positional relationship with the mitral valve. Fig. 4(b) is an end view in which the mitral valve with said ring sewn thereon has been cut along the central axis Z.
[Fig. 5]
   Fig. 5 is a schematic diagram showing a difference between the annuloplasty ring of the present invention and conventional annuloplasty ring in the state of being sewn to a mitral valve annulus. Said ring is shown in Fig. 5(a), and conventional ring is shown in Fig. 5(b).
[Fig. 6]

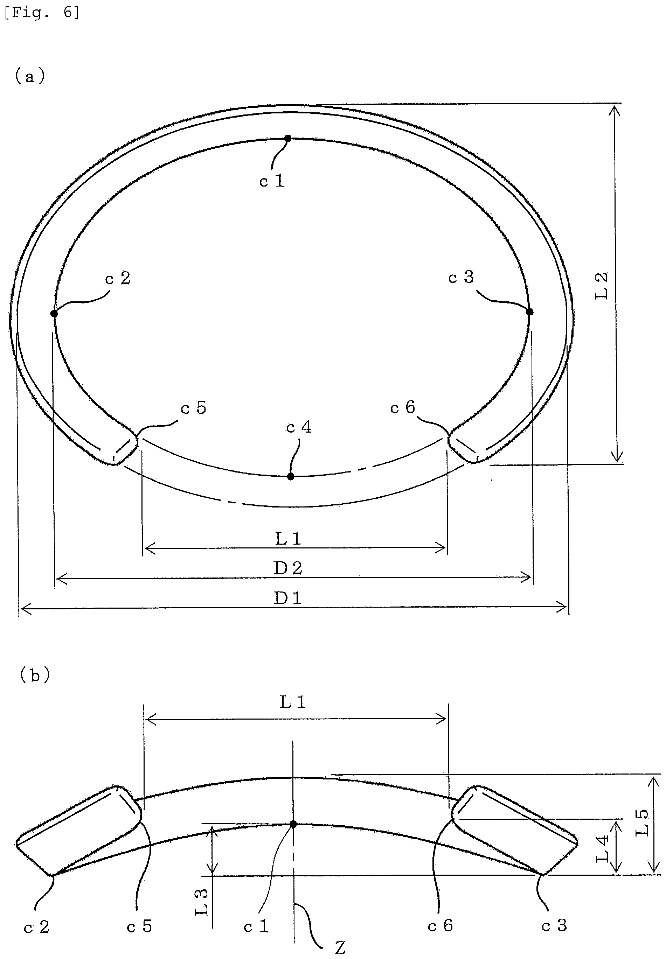

   Fig. 6 is a diagram for illustrating the size of each part of the annuloplasty ring according to the present invention. In Fig. 6(a), said ring is viewed along the central axis Z shown in Fig. 1(b). In Fig. 6(b), said ring shown in Fig. 6(a) is viewed from the downward of Fig. 6(a).
[Fig. 7]

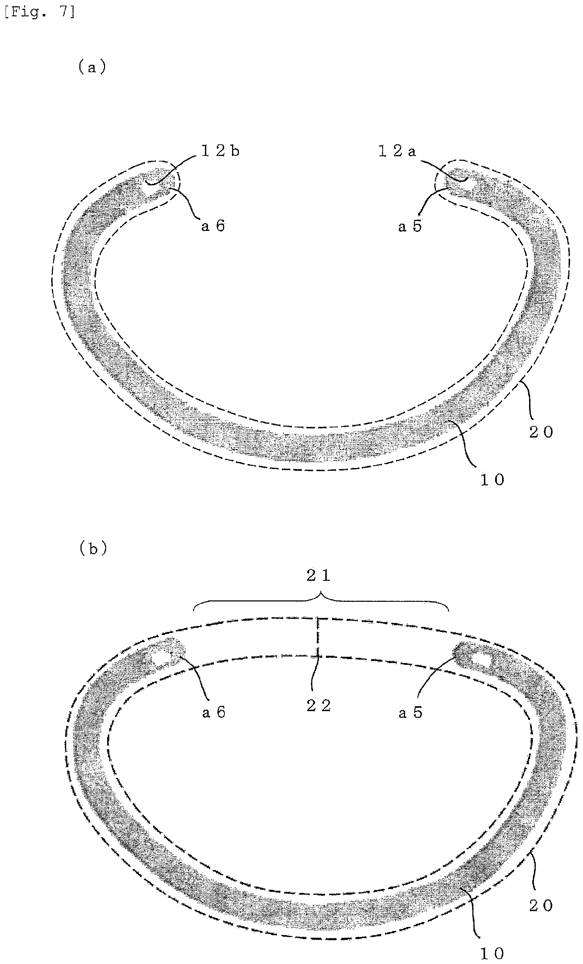

   Fig. 7 shows a preferred embodiment of the annuloplasty ring according to the present invention. In Figs. 7(a) and (b), said ring is viewed along the central axis Z shown in Fig. 1(b). In Figs. 7(a) and (b), the shapes of the core part and the outer layer are shown by superimposing the outline of the core part on top of the outline of said ring.
[Fig. 8]

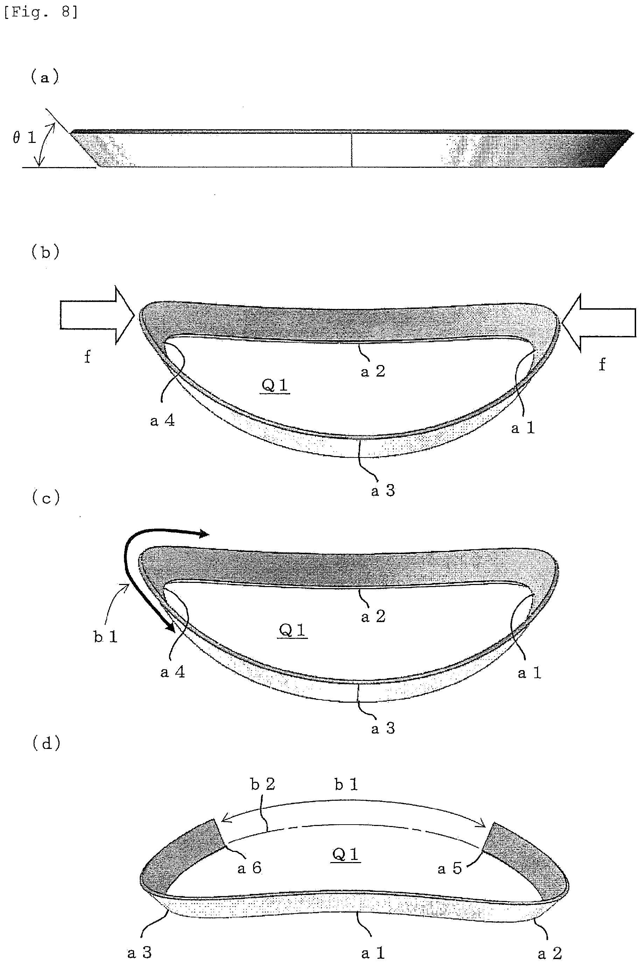

   Fig. 8 shows one example of the production method of the core part of the annuloplasty ring of the present invention.
[Fig. 9]

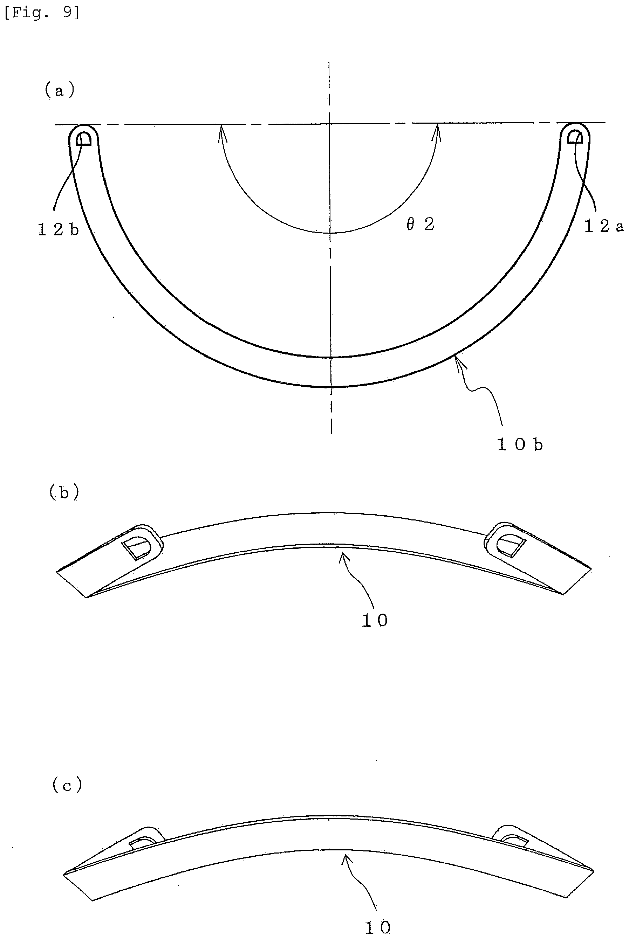

   Fig. 9 shows one example of a preferred production method of the core part of the annuloplasty ring of the present invention. Fig. 9(a) shows one example of the arc-shaped member, and Figs. 9(b) and (c) show one example of a band-shaped plate member (=core part) that is curved to show a C-shape while forming a saddle shape, by deforming the arc-shaped member of Fig. 9(a). In Fig. 9(b), the band-shaped plate member is viewed from the side of the C-shaped notch part. In Fig. 9(c), the band-shaped plate member of Fig. 9(b) is viewed from the side of the back face.
[Fig. 10]

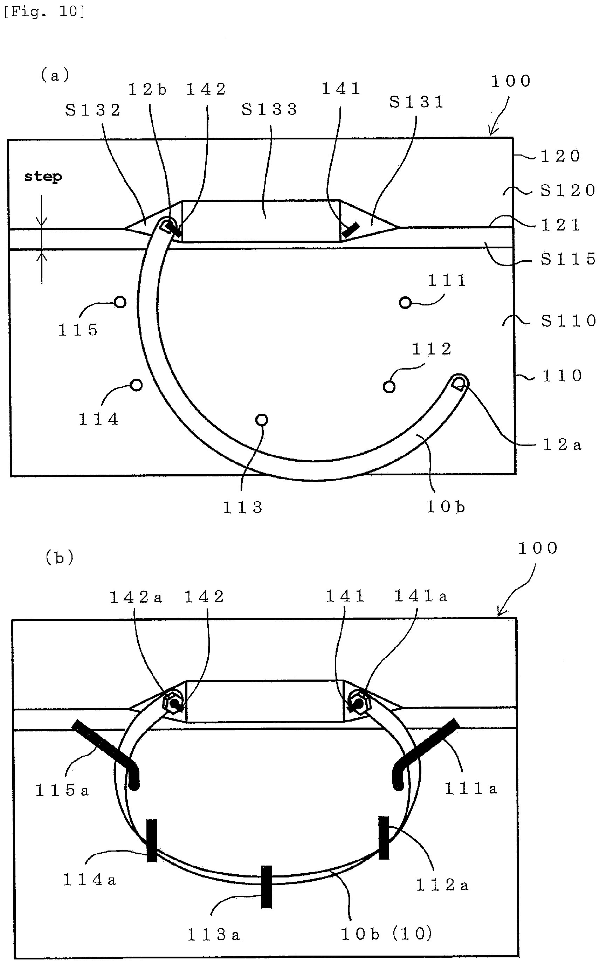

   Fig. 10 is a diagram showing a jig that can preferably perform the production method shown in Fig. 9 and a method of using the jig.
[Fig. 11]

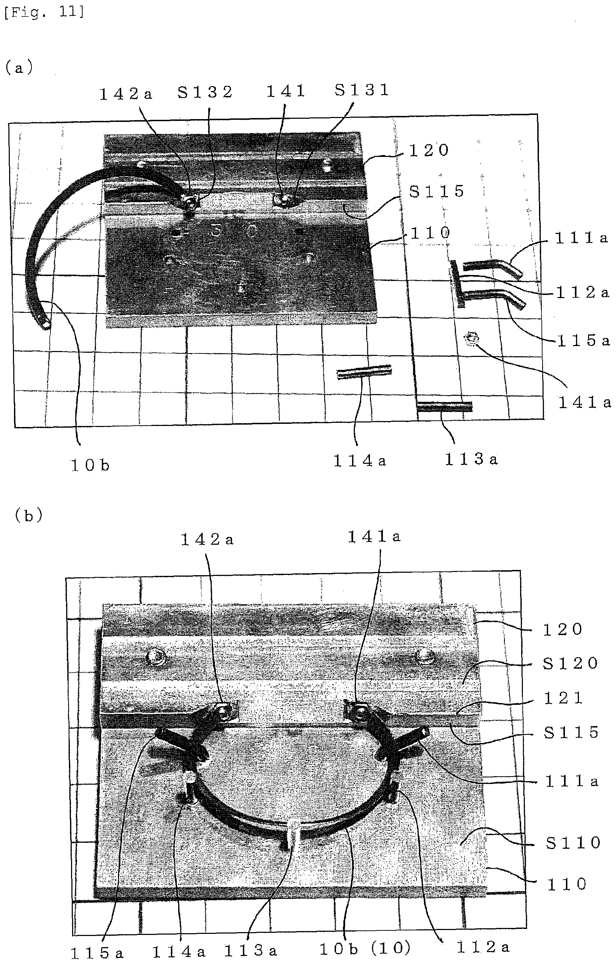

   Fig. 11 shows photographic diagrams of one example of a test product of the jig shown in Fig. 10. In the figure, the jig is placed on a surface with a grid of 10 mm on each side so that the size of the jig can be easily known.
[Fig. 12]
   Fig. 12 shows photographs of examples of conventional mitral annuloplasty rings.
[Fig. 13]
   Fig. 13 is a cut end surface diagram showing a conventional mitral annuloplasty ring in the state of being sewn to a mitral valve annulus.

### [Description of Embodiments]

The annuloplasty ring of the present invention is described first. In the following description, said ring is described using a mitral valve annulus as a valve annulus to mount said ring. Except the description relating to a saddle shape which is a distinct shape of mitral valve annulus, the following description also applies as the description of tricuspid annuloplasty rings. In Figs. 1 to 13, the same symbol is given to the same parts as appropriate, and the same description of those symbols for each figure is omitted.

As shown in one example of the constitution in Fig. 1, said ring has a core part 10 and an outer layer 20 made of a polymer material covering the surface thereof. In Figs. 1(a) and 1(b), a corner line (edge line) is added to said ring for visual clarity. In actual products, the corners are rounded and the edge line may not appear clearly. Although the core part 10 is hidden by the outer layer 20 in Figs. 1(a) and 1(b), the outline of the shape of the core part appears in Figs. 1(a) and 1(b). As shown in the examples of Fig. 1(c), Fig. 8, and Fig. 9, the core part 10 has a structure in which a band-shaped plate member made of a spring material is curved such that the opening part (Q1 in Figs. 8(b) to 8(d)) is defined in a shape along the valve annulus to be the mounting object, and the plate member presents a C-shape.

The band-shaped plate member constituting the core part 10 has a predetermined plate thickness t1 and a predetermined band width w1, as shown in the cross-sectional shape of Fig. 2. Since the plate thickness t1 is sufficiently small with respect to the band width w1, the cross-sectional shape of the band-shaped plate member is an elongated rectangular shape as shown in Fig. 2, and the plate member thus has a longitudinal axis 10a along the long side.

One of the important features of said ring is the shape of the core part. In the present invention, as shown in Fig. 1(c), a designed central axis Z is defined that passes through the opening part defined by the core part in the direction of blood flow (the downward direction indicated by arrow F in the figure). When the core part 10 is cut along a flat plane containing the central axis Z, the cross-sectional shape of the band-shaped plate member is, as described above, an elongated cross-sectional shape as a cross-sectional shape when the plate is cut. In the present invention, it is important that the longitudinal axis 10a of the elongated cross-sectional shape is inclined with respect to the central axis Z. In other words, the band-shaped plate member constituting the core part 10 is inclined to form a funnel shape. As clearly shown in Fig. 1(c), the inclination is such that the diameter of the opening part decreases as the central axis Z moves in the blood flow direction F. In Fig. 1(c), the inclination is defined as an inclination that rises by an angle (elevation angle) Θ1 with respect to a flat plane H1 perpendicular to the central axis Z. Since the core part has such inclination, the cross-section of said ring also has an elongated rectangular shape, as shown in Fig. 1(c), and also has a shape with an inclination such that the diameter of the opening part becomes smaller as the center axis Z moves in the blood flow direction F.

The "blood flow direction" in the definition of said ring is a direction that penetrates the opening part, from the left atrium side to the left ventricle side, assuming that said ring is mounted on the mitral valve annulus. In a preferred embodiment of said ring, the ring has a similar saddle shape to fit the saddle shape formed by a healthy mitral valve annulus itself. Therefore, in Fig. 1(b), the direction in which the two end parts (c5, c6) of the C-shape are raised (upper side of the figure) is the left atrium side, and the opposite side (lower side of the figure) is the left ventricle side.

As shown in Fig. 1 and Fig. 4(a), the saddle shape formed by said ring is a shape in which two curved parts (centers of curvature are c2 and c3) that bend sharply with a larger curvature drop toward the left ventricle side (lower side of the figure), respectively corresponding to the posterior commissure to the vicinity of the right fibrous trigone, and the anterior commissure to the vicinity of the left trigone; a central curved part (center of curvature is c1), which curves more gently with a smaller curvature, gently rises toward the left atrium side, corresponding to the posterior leaflet annulus; and the two ends (c5, c6) also gently rise toward the left atrium side.

By having the above-mentioned constitution, the above-mentioned effects of the invention are obtained, and the above-mentioned problems of the conventional annuloplasty rings are reduced. Each part of said ring is described in more detail in the following.

### (core part)

The core part 10 is a component that generally has a C shape, in which a partial section (section b1 illustrated in Fig. 8(d)) of the entire circumference of the closed loop that can surround a healthy valve orifice (the mitral valve orifice in this example) has been notched. Hereinafter, the part of the notched section b1 in this closed loop is referred to as a "notch part", and the remaining C-shaped part (that is, the substantial part of the core part) is also referred to as the "C-shaped part". Similarly, in said ring, the part of the notched section is called the "notch part", and the substantial part presenting the C shape is also called the "C-shaped part".

### (opening part of core part)

The opening part of the core part 10 is a region defined by the C-shaped part and, in particular, as shown in Fig. 8(d), is a region Q1 defined by the C-shaped curved line formed by the inner edge on the outlet side in the blood flow direction. The C-shaped curved line formed by this inner edge is, in the example of Fig. 8(d), a three-dimensional curve that smoothly passes through one end part a5, an apex a2 of curvature, an apex a1 of curvature (central curved part), an apex a3 of curvature, and the other end part a6 while forming a saddle shape. More particularly, the opening part of the core part 10 is a region surrounded by a closed loop formed by adding the curved line b2 of the notch part to the aforementioned C-shaped curved line. The curved line b2 is a line smoothly curved outward to form a convex, as illustrated in Fig. 8(d). The curved line b2 is smoothly connected to each of the two ends (a5, a6) of the aforementioned C-shaped curved line. Those of ordinary skill in the art with the knowledge of the annular shape formed by a healthy mitral valve annulus can specify the shape of the opening part of the core part 10 by appropriately adding the curved line b2 of the notch part to the C-shaped part of the core part (in particular, the C-shaped curved line formed by the aforementioned inner edge).

### (central axis)

The central axis Z appearing in Figs. 1(b) and 1(c) is a designed standard axis common to the core part and said ring. The central axis Z corresponds to an ideal blood flow axis extending in the direction of blood flow (blood flow direction) through a healthy mitral valve. That is, in the present invention, the "central axis passing through the opening part in the blood flow direction" means, for example, the axis along the blood flow that ideally passes through the center of the opening part when said ring is mounted on the mitral valve annulus. Although it is possible to obtain the central axis Z from the saddle-shaped curve of the core part 10, the curvature of each part of the opening part and the geometrical relationship between the central axis and the opening part become clearer by first determining the central axis Z as a standard line and defining the opening part based thereon. The shape of the opening part and the shape of said ring are described below with the central axis Z as a standard line.

### (shape of said ring)

In Fig. 1(a), said ring is viewed along the central axis Z, from the upstream side (left atrium side, assuming that said ring is mounted on the mitral valve annulus). Hereinafter, this figure is referred to as a top view of said ring. The central axis Z passes through a point P1 perpendicularly to the plane of paper of the top view. The direction of blood flow is along the central axis Z, from the front side to the back side of the plane of paper. As shown in Fig. 1(c), said ring has a structure in which an outer layer 20 is added to the core part 10. Therefore, the shape of said ring in the top view is a C-shape similar to the core part 10. In one example of the present invention, the C-shape appearing in Fig. 1(a) as the top view has a shape close to ellipse, and has a major axis X and a minor axis Y that are perpendicular to the central axis Z at point P1. As shown in Figs. 1(b) and (c), said ring has, similar to core part 10, a funnel-like inner inclined plane S1 and an outer inclined plane S2, and is curved in a C-shape while forming a saddle shape.

### (spring property required for said ring)

The spring properties of said ring are mainly those of the core part. When the outer layer 20 is a solid layer of polymer materials such as an elastomer, the outer layer may also affect the spring properties. When the outer layer 20 is a cloth layer, it does not substantially affect the spring properties. For the spring properties of said ring, reference can be made to the spring properties of conventionally known annuloplasty rings having spring properties, such as the annuloplasty ring shown in Fig. 12(a).

### (materials of band-shaped plate member constituting core part)

For the material of the band-shaped plate member, a springy material that can achieve the above-mentioned dynamic properties required for said ring can be used. Moreover, since said ring is sewn to the mitral valve annulus, the material of the band-shaped plate member needs to have biocompatibility.

The springy material in the present invention includes not only materials exhibiting properties of recovering from deformation as elastic bodies, but also materials exhibiting properties of recovering from deformation as superelastic bodies.

As a springy material that can be used as a material of the band-shaped plate member, a material capable of imparting appropriate springiness (property of being able to elastically return to its original shape even if it is deformed) to the core part as an annuloplasty ring, having biocompatibility, and preferably not prone to change over time can be used. The spring properties of the springy material vary depending on the materials and can be adjusted to appropriate spring properties by locally or entirely changing the thickness and band width of the band-shaped plate member. Examples of such springy material include various plastic materials for medical use (polymer materials permitting elastic deformation and return to the original shape), metal materials and alloy materials with springiness such as β-titanium alloys and Ni-Ti alloys.

### (Ni-Ti alloy as material for band-shaped plate member constituting core part)

Among the above-mentioned materials for the band-shaped plate members, shape memory alloys are preferable materials, and Ni-Ti (nickel-titanium) alloy is a particularly preferred shape memory alloy. In Ni-Ti alloy, for example, "Ti-Ni alloy for implantable applications" specified in JIS T 7404:2013, the mass fraction of Ni is 53.5 to 57.5(%), and the mass fraction of Ti is the balance or 46.5 to 42.5(%).

A preferred Ni-Ti alloy for the core part of said ring is an alloy whose Af point (austenite phase transformation finished temperature) as a shape memory alloy is sufficiently lower than body temperature. A preferable range of such Af point is, for example, not more than 15°C. The lower limit of the Af point is not particularly limited and may be, for example, 0°C. The Af point of the Ni-Ti alloy used in the examples of the present invention is 5°C as designated by the manufacturer, and the Af point was 1 to 8°C in tests according to ASTM F2063-18. With Ni-Ti alloy with such a low Af point, the Ni-Ti alloy expresses superelasticity at general room temperature (20 - 30°C) of a surgery room for performing valvuloplasty, or body temperature of patients (35 - 40°C). For example, even if a relatively large deformation that would cause plastic deformation in general spring materials is applied during surgery, a core part made of the aforementioned Ni-Ti alloy returns to its original shape and can support the valve annulus with appropriate springiness after being sutured to the valve annulus of the patient.

In the Examples of the present invention, a Ni-Ti alloy plate (thickness: 0.3 mm, Ni: 55.85 wt%, Ti: balance) manufactured by Yoshimi Inc. was used as the material for the band-shaped plate member that constitutes the core part. The Ni-Ti alloy plate also contains other trace elements C, O, N, N+O, Co, Cu, Cr, H, Fe, Nb, etc., but the description of trace contents thereof is omitted. The Af point of this Ni-Ti alloy plate as designated by the manufacturer is 5°C as mentioned above, and the Af point was 1 to 8°C in tests according to ASTM F2063-18.

Ni-Ti alloys are sold under the name of, for example, Nitinol (registered trademark), and have conventionally been used in the medical field as a metal material that can be embedded in the body.

In the present invention, a flat C-shaped plate material (arc-shaped member) made of Ni-Ti alloy is deformed using a jig or the like into a saddle-shaped undulating shape that fits a valve annulus, and subjected to a heat treatment including heating the plate in the deformed state to a shape memory heat treatment temperature of about 450 to 520°C (490°C in the Examples) and rapidly cooling same to a temperature below the Af point (e.g. near 0°C for an Af point of 5°C) using a low temperature liquid (e.g., water near 0°C mixed with a sufficient amount of ice). The liquid for heat treatment may be a liquid that does not freeze even at 0°C or lower. By this heat treatment (shape memory treatment), the C-shape with a undulating saddle shape becomes the original shape. The C-shaped and saddle-shaped core part subjected-to the shape memory treatment in this way exhibits preferable springiness as an annuloplasty ring at a temperature inside the human heart (generally about 35 to 40°C including during fever).

The material of the band-shaped plate member may be a single material composed only of the above-mentioned polymer materials or metals (single metal, alloy, shape memory alloy such as Ni-Ti alloy), or a composite material such as a laminate of a layer made of a polymer material and a layer made of a metal material. In addition, the cross-sectional area and cross-sectional shape may be locally changed along the C-shaped curve in order to afford preferred deformation property and stress property.

### (Inclination angle of band-shaped plate member)

As mentioned above, when the core part 10 is cut along a flat plane containing the central axis Z (e.g., any flat plane containing the point P1 in Fig. 1(a) and perpendicular to the plane of paper), the longitudinal axis 10a in the cross-section of the band-shaped plate member is, as shown in Fig. 1(c), inclined by rising by an angle (elevation angle) θ1 with respect to the flat plane H1 perpendicular to the central axis Z. The value of angle θ1 may vary as the C-shaped part moves in the circumferential direction. For example, the value of the angle Θ1 including all variations in angle within one mitral annuloplasty ring and acceptable ranges of the angle θ1 as a mitral annuloplasty ring is generally about 45 to 60 degrees, and 50 degrees is exemplified as a more preferred angle for the mitral valve annulus.

### (thickness and band width of band-shaped plate member)

Fig. 2 is a diagram schematically showing a cross section of said ring cut along a flat plane containing the central axis Z. In the figure, the cross section is shown with the longitudinal axis of the cross section extending in the horizontal direction.

The thickness t1 of the band-shaped plate member, which is the core part 10, varies depending on the material. In the case of Ni-Ti alloy, the thickness is preferably about 0.2 to 0.5 mm, and particularly preferably about 0.3 mm.

The band width w1 of the band-shaped plate member varies depending on the material. In the case of Ni-Ti alloy, it is preferably about 2 to 3 mm. For example, products with different widths may be provided, such as 2.4 mm, 2.6 mm, 2.8 mm, etc., according to the size of the valve annulus of patients.

In the example of Fig. 2, the corner 11 of the cross section of the band-shaped plate member obtained by press punching or laser processing may have a sharp edge or burr. It is preferable to subject such corner to barrel polishing or the like to form a curved surface having suitable roundness.

### (outer layer)

The outer layer is required to be a biocompatible and flexible material. The outer layer may be a solid layer made of a polymer material that can be embedded in the body, such as silicone, polyester, and the like, or a layer of known fabric made of a polymer material that can be embedded in the body (polyester and the like). A cloth outer layer is preferred in that fibroblasts from the valve annulus enter the inside of the porous cloth tissue, and the cell tissue of the valve annulus and the cloth outer layer are integrated. The cloth layer is exemplified by a braid tube made by weaving fibers of polymer materials. Fig. 3 is a photographic view showing one example of the external appearance of the example product of said ring, showing the surface of the braid tube. The end part of cloth layer, such as braid tube, is preferably closed with a suture thread or by heating. For the material and structure of the outer layer, the weaving method of the braid tube, the method of closing the end part, and the like, the outer layers of conventionally known mitral annuloplasty rings can be referred to.

### (preferred embodiment of outer layer)

In a preferred embodiment of said ring, the cross-sectional shape of the internal space of the braid tube used as the outer layer is a rectangle similar to the cross-sectional shape of the band-shaped plate member of the core part (cross-sectional shape shown by hatching in Fig. 2). By using a braid tube having such a cross-sectional shape of the internal space, a phenomenon of rotation of the braid tube around the cross-section of the band-shaped plate member does not occur easily. As a result, a phenomenon in which the braid tube rotates and said ring, which is fixed at a predetermined fixed position on the valve annulus with a suture thread, unintentionally moves from the fixed position can be suppressed.

A braid tube with the aforementioned flat cross-sectional shape can be obtained by braiding threads around a core material having the same cross-sectional shape as the cross-sectional shape of the band-shaped plate member (including those whose size has been slightly adjusted in anticipation of expansion and contraction of the braid tube).

In the present invention, a braid tube with the aforementioned flat cross-sectional shape is defined by a production method, according to the definition, "a braid tube obtained by weaving threads around a core material having the same cross-sectional shape as the above-mentioned cross-sectional shape of the above-mentioned band-shaped plate member". This definition aims to indicate that the internal space of the braid tube has the same cross-sectional shape as that of the band-shaped plate member. This definition uses the production method for definition, based on the fact that it is almost impractical to specify the cross-sectional shape of a flexible and easily deformable braid tube braided with threads.

### (thickness and width of outer layer)

Examples of the size of each part when said ring is a mitral annuloplasty ring are shown below. When said ring is used as a tricuspid annuloplasty ring, the size of each part may be changed as appropriate.

The thickness t2 of the outer layer 20 shown in Fig. 2 varies depending on the material, the tissue of the outer layer, the size of the valve annulus of patient, and the like. It is preferably about 0.3 to 0.8 mm.

### (thickness and width of C-shaped part of said ring)

By suitably selecting and combining the size of the above-mentioned core part and the outer layer, the size of the C-shaped part of said ring shown in Fig. 2 is obtained. For example, the thickness t3 of said ring is preferably about 0.8 mm to 2 mm, more preferably about 1 to 1.2 mm, and is exemplified by 1.1 mm in a preferred example.

In addition, the width w2 of said ring is about 2.8 to 4 mm, and 3 mm is exemplified in a preferred example.

An adhesive layer or other functional layer may be interposed between the core part and the outer layer. From the aspect of safety when embedded in the body, it is preferable to fix the outer layer to the core part with a suture thread without providing an adhesive layer, and to weld the outer layer to the core part by heating necessary parts.

### (size of each part of said ring)

Fig. 6 is a diagram for illustrating the size of each part of said ring. Fig. 6(a) is a top view similar to Fig. 1(a), and Fig. 6(b) is a figure of said ring of Fig. 6(a) seen from the downward of Fig. 6(a), similar to Fig 1(b). By subtracting the thickness of the outer layer (for two layers) from the size shown in the figure, the size of each part of the core part can be obtained. The range of size for each part of said ring shown below is a general example according to the size of the mitral valve annulus of infant to adult patients. According to the exceptions and depending on the application to the tricuspid valve annulus, the size of each part may be changed as appropriate.

In the top view shown in Fig. 6(a), said ring exhibits a shape close to an ellipse when the curved line of the notch part is supplemented as indicated by the dashed line. The two end parts of the C-shape and the section in the vicinity thereof may be locally deformed to follow the shape of the valve annulus. Examples of the size of each part are as follows.

The inner diameter D2 in the major axis direction is the diameter on the ventricle side, and is about 26 to 40 mm. For example, a product lineup that increases by 2 mm, such as 26 mm, 28 mm, 30 mm, 32 mm..., 36 mm, 38 mm, 40 mm, may be provided for physicians and others to select from. The outer diameter D1 in the major axis direction is the diameter on the atrium side, and is obtained by adding twice the size (w2×cos θ1) calculated from the aforementioned width w2 and the inclination Θ1 to the aforementioned inner diameter D2.

The size L1 (linear distance between two ends c5 and c6) of the notch part of said ring is about 20 to 30 mm.

The outer size L2 of the C-shaped part of said ring in the minor axis direction is about 20 to 28 mm.

In the figure shown in Fig. 6(b), each size in the vertical direction of the figure is a size along the direction of the central axis Z. In the following, the size in the vertical direction of Fig. 6(b) is also referred to as "height".

The height difference L3 between the lowest points of the C-shaped part (centers of curvature c2 and c3) and the center of the central curved part of the C-shaped part (center c1 of gentle curve) is about 2.5 to 6 mm. Due to the saddle shape, the lowest points of the C-shaped part are positioned at the peak that is most sunken toward the ventricle side. Here, the height of the lowest points (c2 and c3) is taken to be zero.

The height difference L4 between the lowest points (c2 and c3) of the C-shaped part and the inner (lower) parts (c5 and c6) of the two end parts of the C-shaped part is about 3.2 to 4.4 mm.

The total height L5 of the C-shaped part is obtained by adding the height component (w2×sin(01)) of the width w2 in consideration of the inclination Θ1 to the aforementioned size L3.

The aforementioned size of each part of said ring can be appropriately selected so as to match the size of the mitral valve annulus or tricuspid valve annulus of the patient. It is preferable to prepare in advance said ring in various size stages to meet various valve annulus sizes according to the age, sex, physique, and the like of the patients.

### (other embodiment of C-shape)

In the example of Fig. 1(a), the shape formed by the C-shaped part of said ring is a shape close to an ellipse. In contrast, the example shown in Fig. 7(a) has a preferable shape that is more deformed from an ellipse to better match the actual shape and movement of the mitral valve annulus. This shape ensures that no unpreferable force or deformation is applied to the mitral valve annulus. Similarly for the tricuspid valve annulus, it is preferable to determine the C-shape that said ring forms to better fit the outer circumference shape of the tricuspid valve annulus.

### (preferred embodiment 1)

In a preferred embodiment of said ring, as shown in Fig. 7, through holes 12a and 12b are provided near the end parts of the core part. The through holes 12a and 12b are used as holes for fixing to a jig for forming the core part in the production method described below, and are also used for fixing the core part and the outer layer with threads. The opening shape of the through holes 12a and 12b is not particularly limited, and may be a circle shape or a rectangle for passing a bolt or the like. A semicircle as shown in Fig. 9(a) is more preferred. If it is only used as a hole for fixing to a jig with a bolt or the like, the opening shape of the through hole is preferably a circular shape since it permits passage of male screws and appropriate positioning. However, in order to fix the core part and the outer layer with threads, the threads pass through the through hole many times. As a shape that increases the opening area as much as possible while maintaining an opening shape permitting passage of male screws and appropriate positioning, a semicircular (or D-shaped) opening shape as shown in Fig. Fig. 9(a) can be mentioned.

### (preferred embodiment 2)

In other preferred embodiment of said ring, as shown in Fig. 7(b), both end parts of the outer layer 20 extend into the notch part of the core part 10 (the part between end parts a5 and a6), and the both end parts of the outer layer 20 are joined together at a junction 22 to form a closed loop. As a result, a part 21 composed only of the outer layer without the core part is formed between the end part a5 and the end part a6 of the core part 10. By providing the part 21 of only the outer layer, it is preferably possible to obtain the effect that said ring can be fixed to the anterior leaflet annulus using the part 21.

### (production method)

Next, a method for producing an annuloplasty ring according to the present invention described above will be described through formation of a mitral annuloplasty ring as an example. The production step of the tricuspid annuloplasty ring is basically the same, except for the presence or absence of the saddle shape.

The production method generally has a core part formation step and an outer layer formation step.

### (core part formation step)

A method for producing the core part of said ring can be roughly divided into the following methods (I) and (II).
(I) As shown in Fig. 8(a) to 8(d), a method in which a conical annular ring part (closed loop) made of a band-shaped plate member is formed, then the annular ring part is deformed into a saddle shape, and finally a predetermined section (notch part) is removed to obtain a C-shaped part (core part).
(II) As shown in Fig. 9 to Fig. 11, a method in which a band-shaped plate member that forms an arc on a flat plane is prepared and deformed to obtain a C-shaped part that is inclined conically to form a saddle shape.

In the above-mentioned production method (I), a band-shaped plate having the same material, the same plate thickness, and the same band width as the band-shaped plate member of the above-mentioned core part is processed into a conical annular ring as shown in Fig. 8(a). It is preferable to provide the through holes shown in Fig. 7 in the original band-shaped plate. The processing direction for obtaining the conical annular ring shown in Fig. 8(a) includes, for example, a method of transforming a flat annular ring into a conical shape with a press (in which case no seam is present), and a method of forming a predetermined angle on both end surfaces in the longitudinal direction of a band-shaped plate with a predetermined length to form a loop that exhibits a conical annular ring, and joining the both end surfaces by welding or the like.

Next, as shown in Fig. 8(b), a compressive force f is applied to the aforementioned conical annular ring in the diameter direction to cause deformation into a saddle shape (shape in which two points a1 and a4 are raised with respect to two points a2 and a3 facing each other), and a heat treatment is applied in the deformed state to set the saddle shape as the original shape. When the material of the band-shaped plate member is a Ni-Ti alloy, the shape of the saddle can be memorized with ease.

Next, as shown in Fig. 8(c), a predetermined section b1 including one part a4 from the two raised parts a1 and a4 of the saddle shape is excised to give a core part 10 of interest, as shown in Fig. 8(d). Two end parts a5, a6 are produced by the excision of the predetermined section b1. In the example of Fig. 8, the loop joint is located at a3 for visual clarity, but the joint may be positioned within the predetermined section b1 and removed.

In the production method of the above-mentioned (II), a band-shaped plate member 10b that forms an arc on a flat plane as shown in Fig. 9(a) is first prepared. As the material, a shape memory alloy such as Ni-Ti alloy is preferred. This band-shaped plate member 10b is a member having the same material, the same plate thickness, and the same band width as the band-shaped plate member of the core part 10. The band surface is flat and does not have undulations as in a saddle shape. The central angle θ2 of the arc is about 135 to 228 degrees, and a particularly preferable range is about 170 to 190 degrees. According to the research by the present inventors, a preferred inclination θ1 of the core part shown in Fig. 1(c) is about 50 degrees. If the central angle θ2 of the arc is 180 degrees, θ1 approaches 50 degrees when curved in a C-shape. Therefore, a more preferred value of θ2 is 180 degrees.

The member that forms an arc on a flat plane as shown in Fig. 9(a) can be obtained by punching out from a plate material by pressing, cutting out from a plate material by wire cutting, laser processing, or the like. It is preferable to provide through holes 12a and 12b as shown in Fig. 9 at both end parts of the band-shaped plate member 10b.

Then, the aforementioned band-shaped plate member 10b is further curved and deformed such that the longitudinal axis of the cross section of the band-shaped plate member is inclined with respect to the central axis to afford a core part with a saddle shape as shown in Figs. 9(b) and 9(c), a heat treatment is applied while in the deformed state to give a core part having a curved shape presenting a C-shape while forming a saddle shape as the original shape. When the material of the band-shaped plate member is the above-mentioned Ni-Ti alloy, the aforementioned heat treatment is the above-mentioned shape memory treatment (heating to the shape memory heat treatment temperature and rapid cooling to a temperature lower than the Af point).

The arc-shaped member 10b that forms an arc on a flat plane as shown in Fig. 9(a) can be taken out more from one plate material than a closed annular member, and processing into a C-shaped saddle shape is easier. Thus, the above-mentioned production method (II) is superior to the above-mentioned production method (I).

### (jig preferable for the above-mentioned production method (II))

Fig. 10 shows one example of a jig that can preferably perform the above-mentioned production method (II), and a manner of producing the core part of said ring by using the jig. When the annuloplasty ring to be produced is a mitral annuloplasty ring, and the core part thereof is curved to present a C-shape while forming a saddle shape, the usefulness of the jig is particularly remarkable.

The jig 100 illustrated in this figure has a base part 110 with a first flat plane S110, and a ring-fixing member 120 with a predetermined height is provided at a predetermined position on the first flat plane S110. The ring-fixing member 120 has two inclined planes S131 and S132 described later. These inclined planes aim to fix the two end parts of the arc-shaped member that is deformed into a C-shape while forming a saddle shape. These two inclined planes are at two positions on the first flat plane. When the aforementioned arc-shaped member is bent into the desired C-shape and the two end parts are respectively fixed to the two inclined planes, the two end parts follow the saddle shape. Therefore, these two inclined planes have inclinations at both end parts of the target core part.

The aforementioned two inclined planes S131 and S132 may be provided on one ring-fixing member as in the example of Fig. 10. Alternatively, one inclined plane S131 may be provided on one independent ring-fixing member, and the other inclined plane S132 may be provided on the other independent ring-fixing member. In the example of Fig. 10, the ring-fixing member 120 is one plate member, and two inclined planes S131 and S132 are provided on the corners thereof.

In the example of Fig. 10, both the base part 110 and the ring-fixing member 120 are plate members, and a ring-fixing member 120 is stacked at a predetermined position on the first flat plane S110 of the base part 110 such that these plate members become staircase-like. The ring-fixing member 120 has a step S115 perpendicularly rising from the first flat plane S110. The base part 110 and the ring-fixing member 120 may be a single piece carved from a single material.

Fig. 10 and Fig. 11 are views of the jig 100 obliquely viewed from above so that the first flat plane S110, the stepped plane S115, and the second flat plane S120 can be seen (the front side of the base part 110 is omitted). Fig. 11 shows photographic diagrams showing the prototype of the jig illustrated in Fig. 10 and a manner of producing the core part of said ring by using the prototype.

As clearly shown in Fig. 10(a) and Fig. 11(a), in the jig produced as an example, the edge part 121 where the step surface S115 and the second flat plane S120 intersect is partially scraped off and two inclined planes S131 and S132 are formed to fix the both end parts of the core part to be produced. These inclined planes S131 and S132 have inclination following the inclination of the both end parts of the C-shaped core part exhibiting a saddle shape. The inclined plane S133 is positioned between the two inclined planes S131 and S132 and produced by the formation of the aforementioned two inclined planes S131 and S132. Although the inclined plane S133 is not essential, it is preferable to eliminate the edge part 121 and form the inclined plane S133 for the operation of attaching and detaching the arc-shaped member (core part).

The two inclined planes S131 and S132 each have a fixing part at a height corresponding to the saddle shape. This fixing part is a part for fixing the end part of the arc-shaped member by using through holes 12a and 12b. For example, when the fixing part is a simple female screw, the two end parts of the arc-shaped member can be fixed to the two inclined planes S131 and S132 by tightening bolts or screws. However, when bolts or screws are used for fixing, it is not easy to bend the arc-shaped member and fix same to the fixing part. Therefore, in the jig produced as an example, a screw hole is provided at a right angle to each of the inclined planes S131 and S132 at the center thereof. A bolt is screwed into each screw hole from the back side, and screw parts 141 and 142 of the bolt protrude perpendicularly from each inclined plane. The work of fixing the arc-shaped member to the fixing part while curving same is facilitated by first hooking the through hole at the end part of the arc-shaped member to the screw parts 141 and 142 of the bolt and then tightening the nut.

In the jig illustrated in Fig. 11, the screw parts 141 and 142 of the bolt have a nominal designation of M1.6 (refer to Fig. 10(a) for symbols 141 and 142). Both end parts of the band-shaped plate member 10b can be fixed to the inclined planes S131 and S132 by these screw parts 141 and 142, through holes (e.g., through holes 12a and 12b shown in Fig. 7(a)) provided at both end parts of the band-shaped plate member 10b, and the nuts 141a and 142a. Here, that the center positions of the screw parts 141 and 142 are at positions higher than the first flat plane S110 (predetermined positions according to the saddle shape) is also important in addition to the inclination of the inclined planes S131 and S132. Since the center positions of the screw parts 141 and 142 are at predetermined high positions, the both end parts of the arc-shaped member, bent into a C-shape, are maintained at a high position in a saddle shape along the inclined planes S131 and S132.

On the other hand, as shown in Fig. 10(b), three or more guide members protrude at predetermined positions on the first flat plane S110. In the example of Fig. 10(b), the guide members are five thin round bars (pins) 111a to 115a. The guide member is provided at a position along the C-shape so as to maintain the state of the arc-shaped member deformed into the C-shape forming the aforementioned saddle shape.

In the example of Fig. 10 and Fig. 11, holes 111, 112, 113, 114, and 115 are provided for inserting guide members (pins) to forcibly deform the flat plane arc-shaped member into a three-dimensional saddle shape. Guide members 111a, 112a, 113a, 114a, and 115a are inserted into these holes 111 to 115, respectively, as shown in Fig. 10(b).

Two out of the aforementioned three or more guide members are each located inside the two opposing curved parts that curve at the greatest curvature in the aforementioned C-shape. The two guide members each have a part inclined to the outside of the aforementioned curved part so as to incline the aforementioned arc-shaped member toward the inclined plane of the aforementioned core part.

As shown in Fig. 10(b), the guide members 111a and 115a are located inside the curve at the peak part of the major axis of the ellipse of the core part to be produced, and is inclined toward the outside of the ellipse so as to keep the arc-shaped member (core part 10) oblique. The bent arc-shaped member (core part 10) is regulated from the inside by the inclined parts of the guide members 111a and 115a, pressed so as to be into contact with the first flat plane S110, and is maintained in a preferred ellipse shape.

The remaining guide members other than guide members 111a and 115a among the aforementioned three or more guide members are members that are positioned outside a central curved part of the C-shape to prevent the arc-shaped member from expanding outward, and restrict the shape of the arc-shaped member to the C-shape of the core part of the mitral annuloplasty ring to be produced. The number of members of the remaining guide members is not particularly limited, and may be 1 or 2. For appropriate regulation of the curve of the arc-shaped member with a small number thereof, 3 is the preferred number, as in the guide members 112a, 113a, and 114a shown in Fig. 10(b).

In the examples of Fig. 10(b) and Fig. 11, the guide members 112a, 113a, and 114a are straight pins and, as shown in Fig. 10(b), located outside the gently curved central part including the peak part of the minor axis of the ellipse of the core part 10. The core part 10 is regulated from the outside by the guide members 112a, 113a, and 114a, and maintains a preferred ellipse shape while rising from the first flat plane S110 to form a saddle shape.

Two nuts 141a and 142a are tightened while the core part 10 is curved to form a preferable saddle shape, the core part 10 fixed to the jig 100 is put into a furnace, and a heat treatment (shape memory treatment) is performed so as to make the saddle shape the original shape.

In the jigs shown in Fig. 10 and Fig. 11, only two end parts of the C-shaped core part are immovably fixed, and other parts are capable of slight displacement even though they are restricted by the guide members. When subjected to the heat treatment (shape memory treatment) in such a state, the thermally expanded core part can be displaced relatively freely. Thus, a residual stress due to the heat treatment does not occur easily, and a phenomenon of deformation due to residual stress when released from the jig does not occur easily. Moreover, since the core part is exposed in the furnace, the amount of heat required for the heat treatment advantageously becomes small.

On the other hand, for example, if the entire circumference of the core part is sandwiched between upper and lower metal molds to form a saddle shape, and a heat treatment is performed in this state, there is a high possibility that residual stress will occur since the core part cannot be displaced, and a phenomenon of deformation due to the residual stress may occur when released from the metal mold. In addition, since the core part is confined in the metal mold, a large amount of heat is required for the heat treatment, which is not economical, and the operation of removing the core part from the metal mold and rapidly cooling same is also not easy.

The material of the jig is not particularly limited, and may be carbon steel for machine structure, die steel for metal mold, and the like. The base part 110 and the ring-fixing member 120 are preferably made of a metal material with good thermal conductivity so as not to hinder the heat treatment of the core part. Examples of preferred material include brass, aluminum alloy, stainless steel, and the like. On the other hand, examples of preferred material of the guide member include hard steel materials such as carbon tool steel, die steel, and stainless steel.

### (outer layer formation step)

In the outer layer formation step, the surface of the core part obtained by the aforementioned core part formation step is covered with an outer layer made of a polymer material. The method of covering the surface of the core part with the outer layer may be appropriately selected according to the constitution of the outer layer. When the outer layer is a braid tube made of polyester, the braid tube is, as described above, a so-called hollow cord obtained by braiding around a flat material as a core, which corresponds to the core shape of the ring, and preferably has a flat tubular shape. This is passed through from one end part of the core part and extended to the other end. The fixing of the core part and the outer layer is performed by repeatedly passing a polyester thread through the through hole at the end part of the core part. The end part of the braid tube, which is the outer layer, is closed by ultrasonic welding and then cut off with a knife at a specified distance from the core end part.

### [Industrial Applicability]

According to the present invention, a preferable annuloplasty ring that can be sewn snugly and more stably to the surface of the valve annulus of a patient with a larger contact area than that of conventional products can be provided. As a result, for example, the problem of conventional products that turbulence in the blood flow is generated near the entrance of the mitral valve can be suppressed.

This application is based on a patent application No. 2020-200594 filed in Japan (filing date: December 2, 2020), the contents of which are incorporated in full herein by reference.

### [explanation of symbols]

10 core part
20 outer layer
Z central axis
F blood flow direction
Θ1 angle of inclination of longitudinal axis in the cross section of band-shaped plate member

## Claims

1. An annuloplasty ring comprising a core part and an outer layer made of a polymer material, wherein
the core part has a curved structure in which a band-shaped plate member made of a spring material defines an opening part in a shape along a mitral valve annulus and presents a C-shape,
the outer layer covers a surface of the core part,
the longitudinal axis of the cross section of the band-shaped plate member when the core part is cut along a flat plane containing the central axis passing through the opening part in the blood flow direction is inclined with respect to the central axis, and
the inclination is such that the diameter of the opening part decreases as the central axis moves in the blood flow direction.

2. The annuloplasty ring according to claim 1, wherein the annuloplasty ring is a mitral annuloplasty ring for application to a mitral valve annulus.

3. The annuloplasty ring according to claim 2, wherein the band-shaped plate member is curved so as to present a C-shape while forming a saddle shape, a shape along the mitral valve annulus.

4. The annuloplasty ring according to any one of claims 1 to 3, wherein the material of the band-shaped plate member is an Ni-Ti alloy which is a shape memory alloy.

5. The annuloplasty ring according to claim 4, wherein the Ni-Ti alloy has an Af point of not more than 15°C.

6. The annuloplasty ring according to any one of claims 1 to 5, wherein the longitudinal axis of the cross section of the band-shaped plate member is inclined at an elevation angle of 45 to 60 degrees with respect to a flat plane perpendicular to the central axis.

7. The annuloplasty ring according to any one of claims 1 to 6, wherein the outer layer is a layer made of cloth, the cloth layer is a braid tube obtained by weaving threads around a core material having the same cross-sectional shape as the cross-sectional shape of the band-shaped plate member.

8. The annuloplasty ring according to any one of claims 1 to 7, which is used for treating a heart valve disease.

9. A method for producing the annuloplasty ring according to any one of claims 1 to 8, the method comprising,
(i) a core part formation step that includes preparing an arc-shaped member for forming the core part of the annuloplasty ring, and applying an external force to the arc-shaped member to cause deformation of the core part into a shape of the band-shaped plate member, and heat treating the deformed shape of the band-shaped plate member to form a core part having the shape of the band-shaped plate member as an original shape, wherein
the arc-shaped member is a band-shaped plate having the same material, the same thickness, and the same width as the band-shaped plate member of the core part of the annuloplasty ring, and is curved to form an arc, the plate surface of the band-shaped plate is flat, and the band-width direction of the band-shaped plate coincides with the radial direction of the arc, and
(ii) an outer layer formation step which is a step of covering a surface of the core part with an outer layer made of a polymer material.

10. The production method according to claim 9, wherein the arc-shaped member is a member curved to form an arc with a central angle of 135 to 225 degrees.

11. The production method according to claim 9 or 10, wherein the material of the band-shaped plate member is an Ni-Ti alloy which is a shape memory alloy, and
the heat treatment is rapid cooling from the shape memory heat treatment temperature of the Ni-Ti alloy to a temperature lower than the Af point.

12. The production method according to claim 11, wherein the annuloplasty ring to be produced by the production method is a mitral annuloplasty ring for application to a mitral valve annulus,
a core part of the mitral annuloplasty ring to be produced is curved such that the band-shaped plate member presents a C-shape while forming a saddle shape, a shape along the mitral valve annulus,
each of two end parts of the arc-shaped member is provided with a through-hole for fixing the arc-shaped member in a state of being deformed into the C-shape,
in the core part formation step of the (i) in the production method, a jig is used to hold the arc-shaped member deformed into the C-shape and to perform a heat treatment in that state,
the jig has a base part with a first flat plane, and a ring-fixing member is provided at a predetermined position on the first flat plane,
the ring-fixing member has two inclined planes for fixing the two end parts of the arc-shaped member that is deformed into a C-shape while forming the saddle shape,
the two inclined planes are located at two positions on the first flat plane such that the two end parts of the arc-shaped member are along the saddle shape,
the two inclined planes have the inclination of the core part,
the two inclined planes have a fixing part for fixing the two end parts at a height position corresponding to the saddle shape,
at predetermined positions on the first flat plane, three or more guide members protrude along the C-shape so as to maintain the state of the arc-shaped member deformed into the C-shape forming the saddle shape,
two out of the three or more guide members are each located inside the two opposing curved parts that curve at the greatest curvature in the C-shape, and the two guide members each have a part inclined to the outside of the curved part so as to incline the arc-shaped member toward the inclined plane of the core part, and
the remaining guide members among the three or more guide members are members that are positioned outside a central curved part connecting the two opposing curved parts to prevent the arc-shaped member from expanding outward, and restrict the shape of the arc-shaped member to the C-shape of the core part of the mitral annuloplasty ring to be produced.

13. The production method according to any one of claims 9 to 12, wherein the outer layer is a layer made of cloth, the cloth layer is a braid tube obtained by weaving threads around a core material having the same cross-sectional shape as the cross-sectional shape of the band-shaped plate member.
